# EUROPÄISCHE PATENTSCHRIFT

(11) **EP 1 025 827 B1**
(45) Veröffentlichungstag und Bekanntmachung des Hinweises auf die Patenterteilung: **10.02.2010**
(21) Anmeldenummer: 99123621.7
(22) Anmeldetag: 27.11.1999
(51) Int. Cl.: A61F 13/537, A61F 13/534

(54) **Absorbierender Hygieneartikel zum einmaligen Gebrauch**
Disposable absorbent hygiene article
Article d'hygiène absorbant et jetable

(30) Priorität: 08.02.1999 DE 19905280
(43) Veröffentlichungstag der Anmeldung: 09.08.2000
(73) Patentinhaber: Paul Hartmann Aktiengesellschaft, 89522 Heidenheim (DE)
(72) Erfinder: Malowaniec, Krzysztof Daniel, Dipl.-Ing., 89522 Heidenheim (DE); Oltmann, Eckhard, Dr., 89520 Heidenheim (DE)
(74) Vertreter: Friz, Oliver

(56) Entgegenhaltungen:
- EP-A- 0 689 818
- WO-A-91/11162
- WO-A-99/05999
- DE-A1- 4 338 326
- US-A- 5 294 478

## Beschreibung

Die Erfindung betrifft einen absorbierenden Hygieneartikel zum einmaligen Gebrauch mit einem flüssigkeitsdichten Rückblatt, einem flüssigkeitsdurchlässigen Deckblatt, einem dazwischen angeordneten zur Speicherung von Körperflüssigkeiten, insbesondere von Urin, geeigneten superabsorbierende Polymer-Materialien (SAP-Materialien) enthaltenden Saugkörper und einer flüssigkeitsaufnehmenden Zwischenschicht, die aus einer oberen Lage aus synthetischen Fasern und einer unteren Lage besteht, wobei das durchschnittliche Porenvolumen der oberen Lage größer ist als das durchschnittliche Porenvolumen der unteren Lage der Zwischenschicht und wobei die Zwischenschicht zwischen dem Saugkörper und dem Deckblatt angeordnet ist.

Ein derartiger Hygieneartikel ist aus der DE 92 18 991 U1 bekannt. Ferner ist aus der EP 0 689 818 A2 ein Hygieneartikel bekannt, bei dem die Zwischenschicht zusammen mit der Saugschicht aus einem vielschichtigen Flächenmaterial hergestellt ist, welches ebenfalls die Merkmale des Oberbegriffs des Anspruchs 1 aufweist.

Bei Hygieneartikeln, insbesondere Windeln oder Inkontinenzvorlagen, mit SAP-Materialien im Saugkörper besteht die Gefahr des sogenannten Gelblockings. Im Bereich des Flüssigkeitsanfalls quellen die SAP-Materialien, so dass, insbesondere nach mehrfachem Flüssigkeitsanfall in dem in Rede stehenden Bereich die weitere Flüssigkeitsaufnahme behindert ist. Die zwischen Deckblatt und Saugkörper vorgesehene Zwischenschicht soll daher die auftreffende Flüssigkeit als eine Art Zwischenpuffer aufnehmen innerhalb der Zwischenschicht verteilen und in den Saugkörper ableiten. Auf diese Weise kann Flüssigkeit vom Bereich des Flüssigkeitsanfalls weggeleitet und an davon entfernter Stelle, wo der Saugkörper bzw. die darin enthaltenen SAP-Materialien noch "unbenutzt" zur Verfügung stehen, in diesen eingeleitet werden.

Aus der WO 91/11162 ist ein Hygieneartikel bekannt, bei dem oberhalb des Saugkörpers eine vernetzte Cellulosefasern aufweisende Flüssigkeitserfassungs- und verteilungsschicht vorgesehen ist.

Durch die chemische Vernetzung der Cellulosemoleküle, die wohlgemerkt innerhalb der Cellulosefasern durch Vernetzung der Cellulosemoleküle einer Faser und nicht zwischen den Fasern vorkommt, wird erreicht, dass die Fasern in vergleichsweise steifem, bauschelastischem Zustand vorliegen. Das intramolekulare Absorbtionsvermögen der Fasern ist reduziert. Solche Fasern sind besser als die chemisch nicht vernetzten Cellulosefasern des Saugkörpers geeignet, auch wiederholt auftretende Flüssigkeitsschwälle zu verarbeiten, d.h. schnell aufzunehmen und in sich und in den Saugkörper weiter zu leiten. Bei dem bekannten Hygieneartikel enthält die Zwischenschicht neben den chemisch vernetzten Cellulosefasern ein thermoplastisches Bindematerial, in Form von beigemischten thermoplastischen Fasern. Durch Erhitzen des Fasergemischs werden die thermoplastischen Fasern erschmolzen und fixieren so die chemisch vernetzten Cellulosefasern klebstoffartig miteinander, was als besonders vorteilhaft dargestellt wird.

Die EP 0 397 110 A1 beschreibt eine Windel, bei der die Funktion der vorübergehenden Flüssigkeitsaufnahme durch einer auf synthetischen Fasern basierenden, voluminösen Vliesstoffschicht übernommen wird, die wiederum als Zwischenschicht zwischen Saugkörper und Deckblatt angeordnet ist. Um die Funktion der Zwischenspeicherung und Verteilung der anfallenden Flüssigkeit in befriedigender Weise erfüllen zu können, muss diese Schicht aber ein Flächengewicht von wenigstens 60 g/m² aufweisen. Die Materialien zur Bildung der voluminösen Vliesstoffschicht sind teuer, werfen logistische Probleme auf und lassen sich nur sehr schwer in eine schnelllaufende Maschine integrieren, da eine Endlosrolle dieser Materialien bei endlichen, üblicherweise verwendbaren Durchmessern nur eine sehr begrenzte Lauflänge aufweisen kann, so dass ein häufiger Rollenwechsel die Folge ist.

Ausgehend vom vorstehend beschriebenen Stand der Technik liegt der vorliegenden Erfindung die Aufgabe zugrunde, einen Hygieneartikel mit einem hohen Anteil an superabsorbierenden Polymer-Materialien im Saugkörper bereitzustellen, dessen Speicherkapazität noch besser und vollständiger genutzt werden kann, als dies bei dem aus der WO 91/11162 bekannten Hygieneartikel der Fall ist. Der Hygieneartikel soll desweiteren in wirtschaftlicher Weise herstellbar sein. Ausgehend von den gattungsgemäßen Hygieneartikeln liegt der vorliegenden Erfindung die Aufgabe zugrunde, einen einfach aufgebauten und damit auf wirtschaftliche Weise herstellbaren Hygieneartikel zu schaffen, dessen Flüssigkeitsaufnahmeverhalten und dessen Flüssigkeitshaltevermögen, insbesondere nach mehrmaligem Auftreffen von Flüssigkeit, verbessert ist. Es soll ferner verhindert werden, dass nach mehrfachem Flüssigkeitsanfall ein Benutzer des Hygieneprodukts in der Zwischenschicht verbleibende Flüssigkeit etwa durch den Druck des Körpergewichts an der Haut verspürt.

Diese Aufgabe wird bei einem Hygieneartikel der eingangs erwähnten Art erfindungsgemäß durch die Merkmale des kennzeichnenden Teils des Anspruchs 1 gelöst.

Es wird nach der Erfindung vorgeschlagen, den Faseranteil der unteren Lage der Zwischenschicht ausschließlich aus chemisch vernetzten Cellulosefasern zu bilden und dieser Lage in homogener Vermischung 5-15 Gew.-%, vorzugsweise 8-12 Gew.-% eines superabsorbierenden Polymermaterials zuzugeben. Somit besteht die untere Lage nach der Erfindung aus chemisch vernetzten Cellulosefasern und superabsorbierendem Polymermaterial.
Die positive Wirkung der nach der Erfindung konstruierten zweilagigen Zwischenschicht resultiert aus einem synergistischen Effekt, der sich folgendermaßen erklären lässt: Durch den Gradienten des durchschnittlichen Porenvolumens (betrachtet in Richtung senkrecht zur Ebene des Deckblatts in Richtung auf den Saugkörper) wird erreicht, dass für die auf die obere Lage der Zwischenschicht auftreffende Flüssigkeit, zunächst ein verhältnismäßig großporiges Flüssigkeitsaufnahmevolumen zur Verfügung steht und dass die Flüssigkeit dann über die untere Lage der Zwischenschicht in den Saugkörper gelangen kann. Die geringere Hydrophilität der synthetischen Fasern der oberen Lage der Zwischenschicht gegenüber den chemisch vernetzten Cellulosefasern der unteren Lage der Zwischenschicht und wiederum deren vorzugsweise geringere Hydrophilität gegenüber den nichtvernetzten Cellulosefasern des Saugkörpers unterstützen die gewünschte Richtung des Flüssigkeitstransports. Die aus den chemisch vernetzten Cellulosefasern und superabsorbierendem Polymermaterial bestehende untere Lage der Zwischenschicht weist aufgrund der kapillaren Saugwirkung der geschichteten Fasern ein gewisses Flüssigkeitsleitvermögen auf, so dass durch die obere Lage der Zwischenschicht in die untere Lage gelangte Flüssigkeit in deren Ebene verteilt und somit zum Teil auch in vom Flüssigkeitsanfall beabstandete Bereiche gelangt und dort in den darunter angeordneten Saugkörper abgeführt werden kann. Dennoch wird nach mehrmaligem Auftreffen von Flüssigkeit die Speicherkapazität des Saugkörpers in dem Bereich, in dem die Flüssigkeit auftrifft, nahezu erschöpft sein. In diesem für das Funktionieren des Hygieneartikels kritischen Moment kann die untere Lage der Zwischenspeicherschicht die weiter hinzukommende Flüssigkeit zumindest in beschränktem Ausmaß speichern. Die Speicherkapazität wird zum einen aufgrund des noch vorhandenen molekularen Bindevermögens der vernetzten Fasern und zum anderen durch das zwischen den Fasern verbliebene Porenvolumen und desweiteren durch die Beimischung des Anteils an superabsorbierendem Polymermaterial erreicht. Die untere Lage der Zwischenschicht bietet somit eine Art Reservespeicherkapazität. In der unteren Lage der Zwischenschicht verbliebene Flüssigkeit, die nicht an den darunter angeordneten Saugkörper geleitet wurde, wird durch das superabsorbierenden Polymermaterial aufgenommen und dauerhaft gespeichert. Die Gefahr des Herauspressens von Flüssigkeit an die Windeloberfläche, d.h. an die Haut des Benutzers der Windel, ist auch wesentlich reduziert, da die obere großporige und mit einer noch geringeren Hydrophilität der synthetischen Fasern ausgestattete Lage der Zwischenspeicherschicht als Abstandshalter fungiert. Die synthetischen Fasern der oberen Lage der Zwischenschicht bieten im bevorzugten Fall sehr geringer Hydrophilität kaum Möglichkeit zur molekularen Bindung von Flüssigkeit, so dass diese Schicht weitestgehend trocken bleibt.

Dadurch, dass die obere Lage der Zwischenschicht mit ihren Längsendabschnitten die Längsenden der unteren Lage der Zwischenschicht überlappt, lässt sich sicher vermeiden, dass eventuell in der unteren Lage der Zwischenschicht vorhandene Flüssigkeit an die Oberfläche des Hygieneartikels gedrückt werden kann.

Es hat sich desweiteren als vorteilhaft erwiesen, wenn die Ränder der Längsendabschnitte der oberen Lage noch innerhalb d.h. in einem Abstand von den Längsenden des Saugkörpers liegen. Hierdurch lässt sich die Speicherkapazität des Saugkörpers besser ausnutzen: Wenn im oben beschriebenen Fall des mehrfachen Flüssigkeitsanfalls die Speicherkapazität des Saugkörpers im Auftreffbereich erschöpft ist und die untere Lage der Zwischenspeicherschicht bereits ihre Reservespeicherfunktion aufnimmt und weiter auftreffende Flüssigkeit an der Grenze zwischen den beiden Lagen der Zwischenspeicherschicht bzw. im unteren Bereich der oberen Lage der Zwischenspeicherschicht transportiert wird, so kann die Flüssigkeit bei Erreichen der Längsenden der unteren Lage der Zwischenschicht sicher in den Saugkörper eingeleitet werden, wobei davon ausgegangen wird, dass die Speicherkapazität des Saugkörpers in diesem Bereich noch nicht ausgeschöpft ist.

Der vorstehend erwähnte Flüssigkeitstransport im unteren Bereich der oberen Lage der Zwischenschicht lässt sich noch effektiver gestalten, wenn gemäß einer bevorzugten Ausführungsform die obere Lage ihrerseits ein in Richtung des Flüssigkeitstransports vom Deckblatt zum Saugkörper, also senkrecht zur Schichtebene abnehmendes Porenvolumen aufweist. Das Porenvolumen braucht nicht diskret von einem größeren Wert zu einem kleineren Wert abzunehmen, sondern es kann quasi kontinuierlich innerhalb der oberen Lage der Zwischenschicht von oben nach unten abnehmen.

Bevorzugtermaßen wird die Zwischenschicht so gebildet, dass die Fasern der oberen Lage der Zwischenschicht in die untere Lage der Zwischenschicht eingreifen und somit eine Übergangszone zwischen den Lagen gebildet wird. In entsprechender Weise können die Fasern der unteren Lage der Zwischenschicht in den Saugkörper eingreifen, so dass ebenfalls eine Übergangszone zwischen der Zwischenschicht und dem Saugkörper gebildet wird.

Durch den vorstehend beschriebenen zweischichtigen Aufbau der Zwischenschicht und der Funktionsweise der unteren Lage der Zwischenschicht ist es möglich, das Flächengewicht der oberen Lage der Zwischenschicht gering zu halten, vorzugsweise unterhalb von 55 g/m². Dies eröffnet aber die Möglichkeit, eine Endlosrolle des hierfür benötigten Materials mit einer für den technischen Herstellungsvorgang befriedigend hohen Lauflänge auszustatten und das Material in die schnelllaufende Fertigung zu integrieren.

Zur Bildung der oberen Lage der Zwischenschicht wird vorgeschlagen, einen im Vergleich zum Vliesstoff des Deckblatts voluminösen Vliesstoff zu verwenden. Dem Fachmann stehen eine Reihe von bekannten synthetischen Faserarten sowie Verfahren zur Vliesstoffherstellung zur Verfügung, so dass dies keiner näheren Beschreibung bedarf. Bevorzugt wird der Vliesstoff zumindest anteilig aus ansich bekannten Bikomponentenfasern, z.B. des Kern/Mantel-Typs gebildet. Damit lässt sich ein Bikomponentenfasern enthaltendes Faservlies in bekannter Weise thermisch zum fertigen Vliesstoff konsolidieren, ohne dass die Faserstruktur beeinträchtigt wird. Als vorteilhaft haben sich Faserstärken der Bikomponentenfasern von wenigstens 4 dtex, vorzugsweise von 5 bis 8 dtex erwiesen. Hierdurch wird gewährleistet, dass der Titer der Fasern auch nach der thermischen Konsolidierung noch groß genug ist, um dem Vliesstoff das gewünschte große Porenvolumen zu geben. Dicke Fasern lassen sich nämlich nicht so volumenfüllend packen wie dünne Fasern. Dem Fachmann stehen desweiteren Mittel zur Einstellung der Hydrophilität von synthetischen Fasern zur Verfügung, die daher keiner Erläuterung bedürfen. Unter Verwendung derartiger Mittel lässt sich die vorzugsweise sehr geringe Hydrophilität der oberen Lage der Zwischenschicht so einstellen, dass sie vorzugsweise sehr gering in jedem Fall jedoch geringer als diejenige der chemisch vernetzten Fasern der unteren Lage der Zwischenschicht ist. Es hat sich auch bewährt, den unterhalb der Zwischenschicht angeordneten Saugkörper zweilagig auszubilden, wobei die obere Lage im wesentlichen Cellulosefasern und superabsorbierende Polymer-Materialien und die untere Lage im wesentlichen ausschließlich Cellulosefasern enthält. Hierdurch wird eine optimale Ausnutzung der Saugkapazität des Saugkörpers gewährleistet.

Nach einer weiteren vorteilhaften Ausführungsform der Erfindung wird vorgeschlagen, das Deckblatt integral mit der oberen Lage der Zwischenschicht zu bilden, indem es von einer oberen Teilschicht der oberen Lage der Zwischenschicht gebildet ist. Die obere Lage der Zwischenschicht ist also ihrerseits zweischichtig und kann aus einer oberen Teilschicht aus verhältnismäßig dünnen Fasern mit einer Faserstärke < 2 dtex und aus einer unteren Teilschicht mit Fasern einer Faserstärke > 3,5 dtex bestehen. Ein derartiges Material zur Bildung der oberen Lage der Zwischenschicht ist von der Firma Tenotex (Italien) unter dem Handelsnamen Airten 34 erhältlich.

Weitere Vorteile, Einzelheiten und Merkmale der Erfindung ergeben sich aus den beigefügten Patentansprüchen und der beigefügten zeichnerischen Darstellung und nachfolgenden Beschreibung einer bevorzugten Ausführungsform der Erfindung. In der Zeichnung zeigt:
- Figur 1: eine schematische Darstellung des Aufbaus eines erfindungsgemäßen Hygieneartikels und
- Figur 2: eine Längsschnittansicht der durch die Pfeile II-II bezeichneten Ebene.

Die Figuren zeigen in schematischer Darstellung den Aufbau eines Hygieneartikels in Form einer Windel. Die Windel umfasst ein Deckblatt 2, ein flüssigkeitsundurchlässigen Rückenblatt 4 und einen dazwischen angeordneten Saugkörper 6. Zwischen dem Deckblatt 2 und dem Saugkörper 6 ist eine zweischichtige insgesamt mit dem Bezugszeichen 8 bezeichnete Zwischenschicht vorgesehen. Im dargestellten Fall erstreckt sich das Deckblatt 2 in Längsrichtung 10 der Windel bis zu deren Längsrändern 12 und in Querrichtung 14 bis zu den seitlichen Querrändern 16 der Windel. Im Bereich entlang des Verlaufs der Längsränder 12 und Querränder 16 ist das flüssigkeitsdurchlässige Deckblatt 2 mit dem Rückenblatt 4 durch ansich beliebige und bekannte Fügeverfahren verbunden. Es wird darauf hingewiesen, dass auch eine Ausführungsform denkbar wäre, bei der das Deckblatt 2 in einem Abstand zu den Querrändern 16 endet und dass außerhalb des Deckblatts 2 weitere bspw. bündchenbildende Elemente oder Elastifizierungsmittel vorgesehen werden können.

Die Zwischenschicht 8 ist zweilagig ausgebildet und weist eine obere Lage 18 und eine untere Lage 20 auf. Die obere Lage 18 überlappt mit Längsendabschnitten 19 in Längsrichtung 10 die Längsenden 21 der unteren Lage 20, sie endet aber noch innerhalb bzw. in einem Abstand von Längsenden 22 des Saugkörpers 6. In Querrichtung 14 haben die obere und die untere Lage 18 bzw. 20 dieselbe Breite. Es wäre aber auch denkbar und vorteilhaft, dass die obere Lage 18 die untere Lage 20 auch in Querrichtung überlappt.

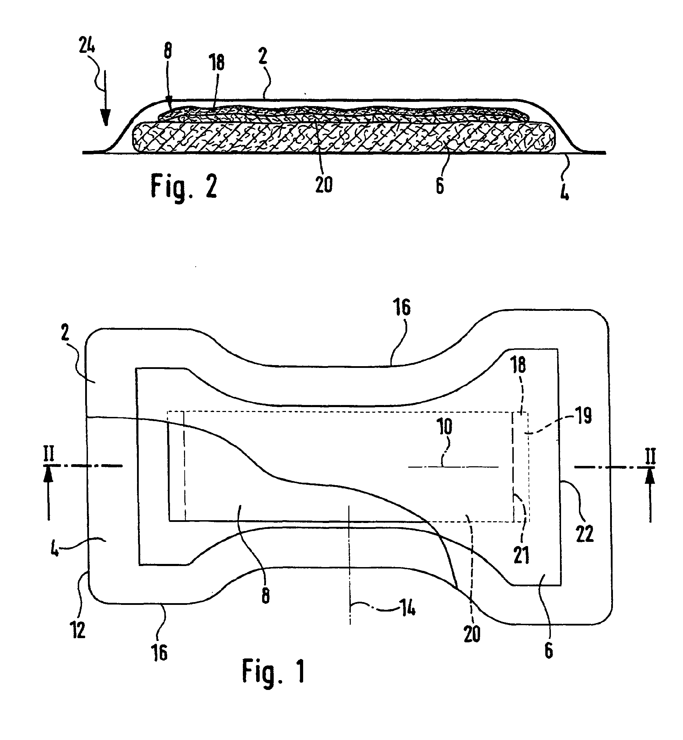

Die obere Lage 18 ist von einem aus synthetischen Fasern bestehenden Vliesstoff gebildet, wobei bevorzugtermaßen ein Anteil von Bikomponentenfasern zum Einsatz kommen. Der Vliesstoff ist im Zuge seiner Herstellung derart verdichtet, dass die obere Lage 18 in Richtung des Pfeils 24 in Figur 2, d.h. in Richtung des Flüssigkeitstransports senkrecht zur flächigen Erstreckung der Zwischenschicht 8, ein abnehmendes Porenvolumen aufweist.

Die untere Lage 20 besteht aus chemisch vernetzten Cellulosefasern, wobei das Porenvolumen der unteren Lage 20 geringer ist als dasjenige der oberen Lage 18 der Zwischenschicht 8. Auch ist die Hydrophilität der chemisch vernetzten Cellulosefasern der unteren Lage 20 größer als diejenige der synthetischen Fasern des die obere Lage 18 bildenden Vliesstoffs.

Bei starkem Flüssigkeitsanfall, wenn also das Flüssigkeitsaufnahmevermögen des Saugkörpers 6 im Bereich des Flüssigkeitsanfalls bereits erschöpft ist, übernimmt die Zwischenschicht 8 eine Flüssigkeitszwischenspeicherund -leitfunktion. Zunächst wird erneut schwallartig anfallende Flüssigkeit in dem verhältnismäßig großporigen Volumen der oberen Lage 18 sehr kurzfristig aufgenommen und dann an die untere Lage 20 der Zwischenschicht 8 abgegeben, womit diese ihre Reservespeicherfunktion ausübt. Ist auch die Speicherkapazität der unteren Lage 20 erschöpft, findet im Bereich des Übergangs der oberen Lage 18 zur unteren Lage 20 eine Flüssigkeitsleitung in der Ebene der flächenhaft erstreckten Lagen statt. Hierdurch wird Flüssigkeit vom Ort der Einleitung wegtransportiert und in im Hinblick auf die Flüssigkeitsaufnahmekapazität noch nicht erschöpfte Bereiche des Saugkörpers 6 in diesen eingeleitet.

## Patentansprüche

1. Absorbierender Hygieneartikel zum einmaligen Gebrauch mit einem flüssigkeitsdichten Rückblatt (4), einem flüssigkeitsdurchlässigen Deckblatt (2), einem dazwischen angeordneten zur Speicherung von Körperflüssigkeiten, insbesondere Urin, geeigneten superabsorbierende Polymer-Materialien enthaltenden Saugkörper (6) und einer zweilagigen flüssigkeitsaufnehmenden und -verteilenden Zwischenschicht (8), die aus einer oberen Lage (18) aus synthetischen Fasern und einer unteren Lage (20) besteht, wobei das durchschnittliche Porenvolumen der oberen Lage (18) größer ist als das durchschnittliche Porenvolumen der unteren Lage (20) der Zwischenschicht (8) und wobei die Zwischenschicht zwischen dem Saugkörper (6) und dem Deckblatt (2) angeordnet ist, **dadurch gekennzeichnet, dass** der Faseranteil der unteren Lage (20) aus chemisch vernetzten Cellulosefasern gebildet ist und die untere Lage (20) ferner 5-15 Gew.-%, vorzugsweise 8-12 Gew.-% eines superabsorbierenden Polymermaterials enthält und die obere Lage (18) mit ihren Längsendabschnitten (19) die Längsenden (21) der unteren Lage (20) überlappt und dass die Hydrophilität der chemisch vernetzten Cellulosefasern der unteren Lage (20) größer ist als die der synthetischen Fasern der oberen Lage (18) der Zwischenschicht (8).

2. Hygieneartikel nach Anspruch 1, **dadurch gekennzeichnet, dass** die Längsenden der oberen Lage (18) der Zwischenschicht (8) von den Längsenden (22) des Saugkörpers beabstandet sind.

3. Hygieneartikel nach einem der vorstehenden Ansprüche, **dadurch gekennzeichnet, dass** die obere Lage (18) durch einen Bikomponentenfasern enthaltenden Vliesstoff gebildet ist.

4. Hygieneartikel nach Anspruch 3, **dadurch gekennzeichnet, dass** die Bikomponentenfasern eine Faserstärke von mindestens 4 dtex, vorzugsweise von 5 bis 8 dtex, aufweisen.

5. Hygieneartikel nach einem der vorstehenden Ansprüche, **dadurch gekennzeichnet, dass** die flächenbezogene Masse des die obere Lage (18) der Zwischenschicht (8) bildenden Vliesstoffs kleiner als 55 g/m² ist.

6. Hygieneartikel nach einem der vorstehenden Ansprüche, **dadurch gekennzeichnet, dass** die durchschnittliche Porengröße der unteren Lage (20) der Zwischenschicht (8) größer ist als die des Saugkörpers (6).

7. Hygieneartikel nach einem der vorstehenden Ansprüche, **dadurch gekennzeichnet, dass** das Porenvolumen der oberen Lage (18) der Zwischenschicht (8) in Richtung senkrecht zur unteren Lage (20) abnimmt.

8. Hygieneartikel nach einem der vorstehenden Ansprüche, **dadurch gekennzeichnet, dass** die obere Lage (18) der Zwischenschicht (8) eine das Deckblatt bildende obere Teilschicht und eine untere Teilschicht aufweist, sodass das Deckblatt integral mit der Zwischenschicht ausgebildet ist, wobei das Porenvolumen der oberen Teilschicht größer ist als das der unteren Teilschicht.

9. Hygieneartikel nach einem der vorstehenden Ansprüche, **dadurch gekennzeichnet, dass** die Fasern der oberen Lage (18) der Zwischenschicht (8) in die untere Lage (20) der Zwischenschicht (8) eingreifen, so dass eine Übergangszone zwischen den Lagen (18, 20) gebildet.

10. Hygieneartikel nach einem der vorstehenden Ansprüche, **dadurch gekennzeichnet, dass** die Fasern der unteren Lage (20) der Zwischenschicht (8) in den Saugkörper (6) eingreifen, so dass eine Übergangszone zwischen der Zwischenschicht (8) und dem Saugkörper (6) gebildet wird.

11. Hygieneartikel nach einem der vorstehenden Ansprüche, **dadurch gekennzeichnet, dass** der Saugkörper zweilagig ist, wobei die obere Lage im wesentlichen Cellulosefasern und superabsorbierendes Polymer enthält und die untere Lage im wesentlichen ausschließlich Cellulosefasern enthält.

12. Hygieneartikel nach einem der vorstehenden Ansprüche, **dadurch gekennzeichnet, dass** das Deckblatt integraler Bestandteil der oberen Lage der Zwischenschicht ist, indem das Deckblatt von einer oberen Teilschicht der oberen Lage der Zwischenschicht gebildet ist.

## Claims

1. Absorbent disposable hygiene article for once-only use with a rear sheet (4), which is impermeable to liquid, a cover sheet (2), which is permeable to liquid, an intermediately arranged absorbent body (6) containing suitable super-absorbent polymer materials for the retention of body fluids, in particular urine and a two-ply liquid-absorbing and distributing intermediate layer (8), which comprises an upper ply (18) of synthetic fibres and a lower ply (20), whereby the average pore volume of the upper ply (18) is greater than the average pore volume of the lower ply (20) of the intermediate layer (8) and whereby the intermediate layer is arranged between the absorbent body (6) and the cover sheet (2), **characterised in that** the fibre portion of the lower ply (20) is formed from chemically cross-linked cellulose fibres and in addition the lower ply (20) contains 5-15% by weight and preferably 8-12% by weight of a super-absorbent polymer material and with its longitudinal end portions (19) the upper ply (18) overlaps the longitudinal ends (21) of the lower ply (20) and that the hydrophility of the chemically cross-linked cellulose fibres of the lower ply (20) is greater than that of the synthetic fibres of the upper ply (18) of the intermediate layer (8).

2. Hygiene article according to claim 1, **characterised in that** the longitudinal ends of the upper ply (18) of the intermediate layer (8) are separated from the longitudinal ends (22) of the absorbent body.

3. Hygiene article according to one of the foregoing claims, **characterised in that** the upper ply (18) is formed by a nonwoven fabric containing two-component fibres.

4. Hygiene article according to claim 3, **characterised in that** the two-component fibres have a fibre size of at least 4 dtex and preferably from 5 to 8 dtex.

5. Hygiene article according to one of the foregoing claims, **characterised in that** the surface area-based mass of the nonwoven fabric forming the upper ply (18) of the intermediate layer (8) is less than 55 g/m².

6. Hygiene article according to one of the foregoing claims, **characterised in that** the average pore size of the lower ply (20) of the intermediate layer (8) is greater than that of the absorbent body (6).

7. Hygiene article according to one of the foregoing claims, **characterised in that** the pore volume of the upper ply (18) of the intermediate layer (8) reduces in a direction at right angles to the lower ply (20).

8. Hygiene article according to one of the foregoing claims, **characterised in that** the upper ply (18) of the intermediate layer (8) has an upper part layer, which forms the cover sheet and a lower part layer, such that the cover sheet is formed integral with the intermediate layer, whereby the pore volume of the upper part layer is greater than that of the lower part layer.

9. Hygiene article according to one of the foregoing claims, **characterised in that** the fibres of the upper ply (18) of the intermediate layer (8) engage in the lower ply (20) of the intermediate layer (8), such that a transition zone is formed between the plies (18, 20).

10. Hygiene article according to one of the foregoing claims, **characterised in that** the fibres of the lower ply (20) of the intermediate layer (8) engage in the absorbent body (6), such that a transition zone is formed between the intermediate layer (8) and the absorbent body (6).

11. Hygiene article according to one of the foregoing claims, **characterised in that** the absorbent body is of two-ply construction, whereby the upper ply essentially contains cellulose fibres and super-absorbent polymer and the lower ply substantially exclusively contains cellulose fibres.

12. Hygiene article according to one of the foregoing claims, **characterised in that** the cover sheet is an integral part of the upper ply of the intermediate layer, **in that** the cover sheet is formed from an upper part layer of the upper ply of the intermediate layer.

## Revendications

1. Article d'hygiène absorbant à usage unique comprenant une feuille arrière étanche aux liquides (4), une feuille de couverture perméable aux liquides (2), un corps absorbant (6) agencé entre les deux et contenant des matériaux polymères superabsorbants et destiné à stocker des liquides corporels, notamment de l'urine, et comprenant une couche intermédiaire (8) à deux couches destinée à recevoir et à répartir le liquide, laquelle couche intermédiaire est composée d'une couche supérieure (18) en fibres synthétiques et d'une couche inférieure (20), le volume poreux moyen de la couche supérieure (18) étant supérieur au volume poreux moyen de la couche inférieure (20) de la couche intermédiaire (8) et la couche intermédiaire étant agencée entre le corps absorbant (6) et la feuille de couverture (2), **caractérisé en ce que** la part en fibres de la couche inférieure (20) est constituée de fibres de cellulose réticulées chimiquement, **en ce que** la couche inférieure (20) contient en outre de 5 à 15 % en poids, de préférence de 8 à 12 % en poids d'un matériau polymère superabsorbant, **en ce que** la couche supérieure (18), avec ses segments d'extrémité longitudinale (19) vient recouvrir les extrémités longitudinales (21) de la couche inférieure (20) et **en ce que** l'hydrophilité des fibres de cellulose réticulées chimiquement de la couche inférieure (20) est supérieure à celle des fibres synthétiques de la couche supérieure (18) de la couche intermédiaire (8).

2. Article d'hygiène selon la revendication 1, **caractérisé en ce que** les extrémités longitudinales de la couche supérieure (18) de la couche intermédiaire (8) sont situées à distance des extrémités longitudinales (22) du corps absorbant.

3. Article d'hygiène selon l'une des revendications précédentes, **caractérisé en ce que** la couche supérieure (18) est constituée d'un non-tissé contenant des fibres à deux composants.

4. Article d'hygiène selon la revendication 3, **caractérisé en ce que** les fibres à deux composants présentent une grosseur de fibres d'au moins 4 dtex, de préférence de 5 à 8 dtex.

5. Article d'hygiène selon l'une des revendications précédentes, **caractérisé en ce que** la masse rapportée à la surface du non-tissé constituant la couche supérieure (18) de la couche intermédiaire (8) est inférieure à 55 g/m².

6. Article d'hygiène selon l'une des revendications précédentes, **caractérisé en ce que** la grosseur de pore moyenne de la couche inférieure (20) de la couche intermédiaire (8) est supérieure à celle du corps absorbant (6).

7. Article d'hygiène selon l'une des revendications précédentes, **caractérisé en ce que** le volume poreux de la couche supérieure (18) de la couche intermédiaire (8) diminue au fur et à mesure que l'on se rapproche de la couche inférieure (20).

8. Article d'hygiène selon l'une des revendications précédentes, **caractérisé en ce que** la couche supérieure (18) de la couche intermédiaire (8) présente une sous-couche supérieure formant la feuille de couverture et une sous-couche inférieure de sorte que la feuille de couverture est réalisée d'un seul tenant avec la couche intermédiaire, le volume poreux de la sous-couche supérieure étant supérieur à celui de la sous-couche inférieure.

9. Article d'hygiène selon l'une des revendications précédentes, **caractérisé en ce que** les fibres de la couche supérieure (18) de la couche intermédiaire (8) pénètrent dans la couche inférieure (20) de la couche intermédiaire (18) de façon à former une zone de transition entre les couches (18, 20).

10. Article d'hygiène selon l'une des revendications précédentes, **caractérisé en ce que** les fibres de la couche inférieure (20) de la couche intermédiaire (8) pénètrent dans le corps absorbant (6) de façon à former une zone de transition entre la couche intermédiaire (8) et le corps absorbant (6).

11. Article d'hygiène selon l'une des revendications précédentes, **caractérisé en ce que** le corps absorbant est lui aussi constitué de deux couches, la couche supérieure contenant essentiellement des fibres de cellulose et un polymère superabsorbant et la couche inférieure contenant exclusivement des fibres de cellulose.

12. Article d'hygiène selon l'une des revendications précédentes, **caractérisé en ce que** la feuille de couverture fait partie intégrante de la couche supérieure de la couche intermédiaire, la feuille de couverture étant constituée d'une sous-couche supérieure de la couche supérieure de la couche intermédiaire.
